# EUROPEAN PATENT APPLICATION

(11) **EP 4 318 438 A1**
(43) Date of publication of application: **07.02.2024**
(21) Application number: 22780880.5
(22) Date of filing: 29.03.2022
(51) Int. Cl.: G09B 9/00, G09B 19/24, G09B 23/28, A61M 25/00

(54) **CATHETER SIMULATOR AND CEREBROVASCULAR MODEL**

(30) Priority: 01.04.2021 JP 2021062691; 26.05.2021 JP 2021088322
(71) Applicant: OSAKA UNIVERSITY, Suita-shi Osaka 565-0871 (JP); JMC Corporation, Yokohama-shi, Kanagawa 222-0033 (JP)
(72) Inventor: OKAYAMA, Keita, Suita-shi, Osaka 565-0871 (JP); SAKATA, Yasushi, Suita-shi, Osaka 565-0871 (JP); WATANABE, Daichi, Yokohama-shi, Kanagawa 222-0033 (JP); INADA, Makoto, Yokohama-shi, Kanagawa 222-0033 (JP)
(74) Representative: advotec.
(86) International application number: PCT/JP2022/015338
(87) International publication number: WO 2022/210669

(57) **Abstract**

Provided is a catheter simulator capable of improving catheter maneuvers for brain diseases with a simple configuration. The catheter simulator has: a container (10); a cerebral blood vessel model (100) held in the container in a state of accommodating a liquid; and a holding means provided on side walls of the container and the cerebral blood vessel model and holding the cerebral blood vessel model in a state of having the container filled with the liquid. The holding means includes a first holding part (21) for holding an end of an ascending aorta (101a) of the cerebral blood vessel model, and a second holding part (22) for holding an end of a descending aorta (101b) of the cerebral blood vessel model, the first holding part (21) includes a liquid introduction port for introducing a liquid into the cerebral blood vessel model, the second holding part (22) includes a catheter introduction port for introducing a catheter into the cerebral blood vessel model, and opening parts (121, 122) for controlling the balance of the liquid circulating inside the cerebral blood vessel model are formed in the outer shell (100A) constituting the cerebral blood vessel model.

## Description

### TECHNICAL FIELD

The present invention relates to a catheter simulator and a cerebral blood vessel model used therein.

### BACKGROUND ART

In recent years, surgeries using catheters have been performed for heart diseases and brain diseases. Catheter surgery is generally performed by inserting a catheter through an artery in an arm or a leg, causing the catheter to reach an affected area, and performing various treatments. With regard to such catheter maneuvers, various catheter simulators (hereinafter, also referred to as simulators) have been proposed to promote acquisition and mastery of the manipulation techniques. For example, in Patent Document 1, a simulator with which a blood vessel model is installed in a human mannequin body, a catheter is introduced into this blood vessel model, and a stent for vasodilation is installed, and a simulator that allows practice of the maneuvers related to coil packing for occlusion intended for preventing rupture of an aneurysm, have been disclosed.

However, in simulators such as described above, since a blood vessel model is installed inside a human mannequin body, the simulator itself tends to be large-sized. For this reason, storage, transportation, as well as preparation and cleanup are not easy, and practice cannot be carried out conveniently.

Thus, the inventors of the present invention have proposed a simulator in which a heart model is installed in a floating state in a container filled with a liquid such as water, and an inflowing port for allowing the liquid to flow in from a pump, and an outflow port for discharging the liquid inside the container toward the pump are formed in the side walls of the container, to circulate the liquid inside the heart model (Patent Document 2). In such a simulator, an introduction part for catheter introduction is formed in the side wall of the container, a pulsatile flow is circulated in the heart model by a pump, and training of the catheter manipulation can be carried out with a simple configuration. In this case, the heart model is caused to pulsate (periodic contraction motion) by adjusting the power output of the pump, and the catheter maneuvers for the heart model can be practiced in a more realistic state.

### CITATION LIST

### PATENT DOCUMENT

Patent Document 1: JP 2014-228803 A
Patent Document 2: JP 6452715 B2

### SUMMARY OF THE INVENTION

### PROBLEM TO BE SOLVED BY THE INVENTION

The simulator disclosed in Patent Document 2 as described above has a simple configuration; however, the simulator is aimed at a heart model imitating an actual heart so that catheter maneuvers for heart diseases can be practiced. Accordingly, the simulator cannot be utilized as a simulator for catheter maneuvers for brain diseases (blood vessels in the brain of the human body).

In an actual human body, blood flow to the brain is sent, from the heart via the aorta, through the left and right carotid arteries and the left and right vertebral arteries, and in the cranium, a large number of blood vessels branch off from these four blood vessels. Generally, examples of a brain disease that requires catheter surgery include cerebral aneurysm and carotid artery stenosis, and catheter surgery is achieved by performing a manipulation of inserting a catheter mainly through an artery at the groin (femoral artery) or a blood vessel of a hand or an arm (radial artery or brachial artery) and guiding the catheter to the target site. Specifically, a contrast medium is caused to flow into the blood vessel, X-ray fluoroscopy is performed, and the catheter is guided to the target site while being observed from an enlarged image thereof. Then, a microcatheter that has been inserted into the catheter is caused to reach the target site, and predetermined medical treatment (surgical clipping, coil embolization, or the like) is carried out by manipulating the microcatheter.

When such catheter maneuvers for brain diseases are practiced, it is necessary to install a dedicated cerebral blood vessel model in the container of the simulator disclosed in Patent Document 2; however, the specific configuration, mode of installation, and the like of the cerebral blood vessel model are not disclosed. Therefore, the simulator cannot be used for practicing the catheter maneuvers for brain diseases. In addition, in the pump that generates a pulsatile flow as disclosed in Patent Document 2, even when the power output is adopted as it is into the cerebral blood vessel model, the circulation balance of blood in the blood vessels is not appropriate, and the pump does not allow hands-on practice.

The present invention was achieved in view of the circumstances as described above, and it is an object of the invention to provide a catheter simulator that enables improvement of catheter maneuvers for brain diseases with a simple configuration, and a cerebral blood vessel model used in the catheter simulator.

### MEANS FOR SOLVING PROBLEM

In order to achieve the above-described object, the catheter simulator according to the present invention has: a container capable of accommodating a liquid in an accommodation part surrounded by side walls and a bottom part; a cerebral blood vessel model held in the container in a state of accommodating a liquid; a pump connected to the container and circulating the liquid inside the cerebral blood vessel model held therein; and a holding means provided on the side walls of the container and the cerebral blood vessel model and holding the cerebral blood vessel model in a state of having the container filled with the liquid, in which the holding means includes a first holding part holding an end of an ascending aorta of the cerebral blood vessel model, and a second holding part holding an end of a descending aorta of the cerebral blood vessel model, the first holding part includes a liquid introduction port for introducing the liquid into the cerebral blood vessel model in the state of being held, the second holding part includes a catheter introduction port for introducing a catheter into the cerebral blood vessel model in the state of being held, and an opening part for controlling the balance of the liquid circulating inside the cerebral blood vessel model by discharging the liquid to be introduced by the pump is formed on an outer shell constituting the cerebral blood vessel model.

In the above-described catheter simulator, a blood flow similar to the actual human body can be created by producing a cerebral blood vessel model having the same size as the actual human body, installing this model in the container, filling the container with a liquid, and circulating the liquid inside the model through a pump. By inserting a catheter through the catheter introduction port into the cerebral blood vessel model installed in this way, the catheter maneuvers can be practiced with a simple configuration. In this case, in the actual human body, since about 15% of the blood flow sent from the heart circulates in the brain while about 90% of the blood flow circulates in the body, the balance of the liquid circulating in the blood vessels in the brain can be adjusted by forming an opening part in the outer shell constituting the cerebral blood vessel model, and it is possible to perform simulation of the catheter maneuvers in a state close to the blood flow in the actual human body.

In addition, in order to achieve the above-described object, according to the present invention, on the occasion of providing a cerebral blood vessel model that is held in a container accommodating a liquid and is used when catheter maneuvers are practiced in a state of having the liquid circulated inside the model, an opening part for controlling the balance of the circulating liquid is formed in the outer shell constituting the cerebral blood vessel model.

According to such a cerebral blood vessel model, when the model is installed in the container of a catheter simulator, and the liquid is circulated inside the model, the flow of the liquid can be controlled through the opening part formed in the outer shell, and therefore, it is possible to perform a simulation of the catheter maneuvers in a state close to the blood flow in the actual human body.

### EFFECT OF THE INVENTION

According to the present invention, there are obtained a catheter simulator capable of improving catheter maneuvers for brain diseases with a simple configuration, and a cerebral blood vessel model used in this catheter simulator.

### BRIEF DESCRIPTION OF DRAWINGS

Fig. 1 is a diagram illustrating an embodiment of a catheter simulator according to the present invention;
Fig. 2 is a plan view illustrating a container of the catheter simulator shown in Fig. 1;
Fig. 3 is a diagram illustrating a configuration example of a cerebral blood vessel model installed in the container of the catheter simulator shown in Fig. 1;
Fig. 4 is a plan view illustrating a state in which the cerebral blood vessel is installed in the container shown in Fig. 2;
Fig. 5 is a diagram viewing the state in which the cerebral blood vessel is installed in the container shown in Fig. 2, from one lateral face side;
Fig. 6 is a cross-sectional view illustrating an example of making the blood vessel parts of the cerebral blood vessel model exchangeable;
Fig. 7 is a diagram illustrating a first example of a flow rate adjustment means for the liquid flowing into the cerebral blood vessel model;
Fig. 8 is a cross-sectional view taken along line A-A in Fig. 7;
Fig. 9 is a diagram illustrating a second example of the flow rate adjustment means for the liquid flowing into the cerebral blood vessel model;
Fig. 10 is a diagram illustrating a third example of the flow rate adjustment means for the liquid flowing into the cerebral blood vessel model;
Fig. 11 is a diagram illustrating a modification example of a blood vessel group of the cerebral blood vessel model; and
Fig. 12 is a diagram illustrating a second modification example of the blood vessel group of the cerebral blood vessel model.

### MODE(S) FOR CARRYING OUT THE INVENTION

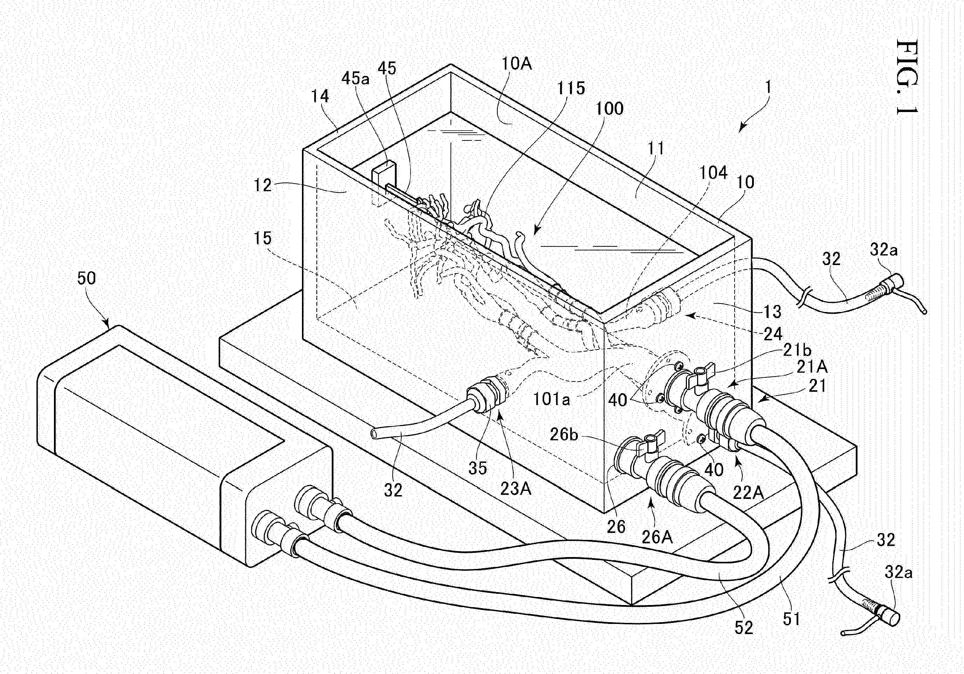

Hereinafter, embodiments of the catheter simulator and the blood vessel model according to the present invention will be described with reference to the attached drawings, by referring to Fig. 1 to Fig. 5.

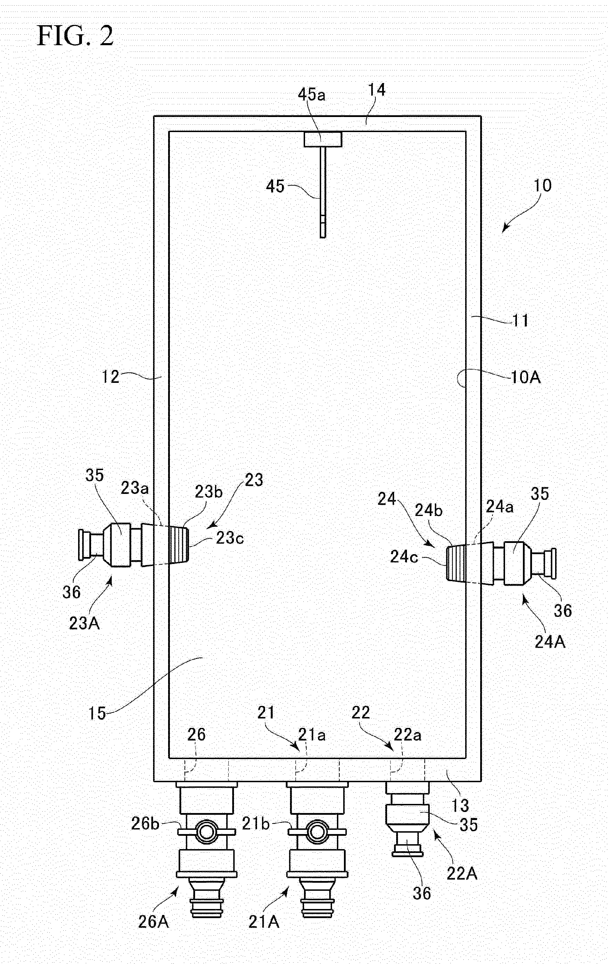

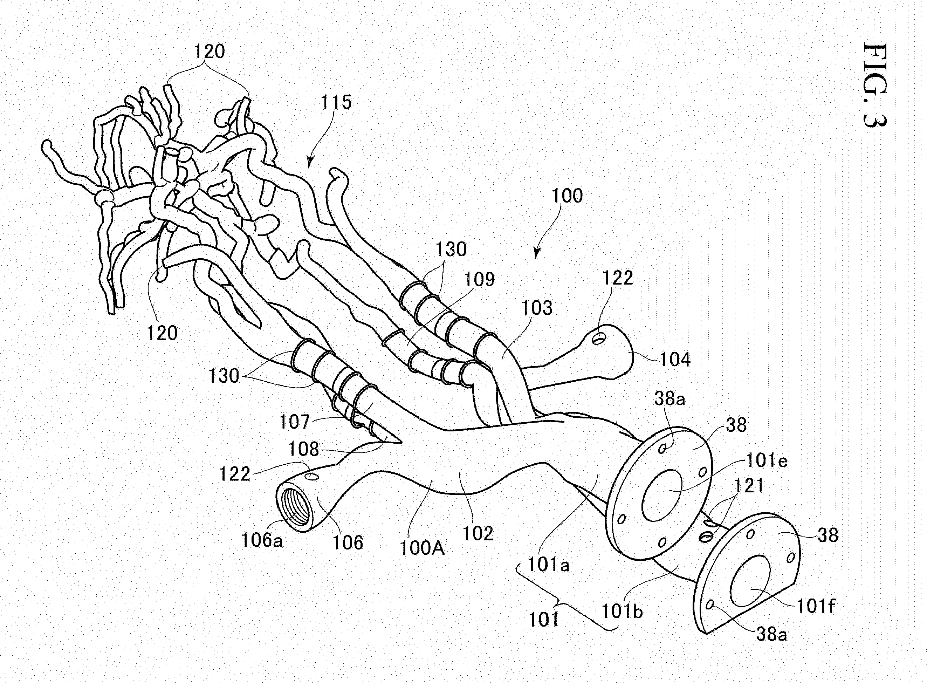

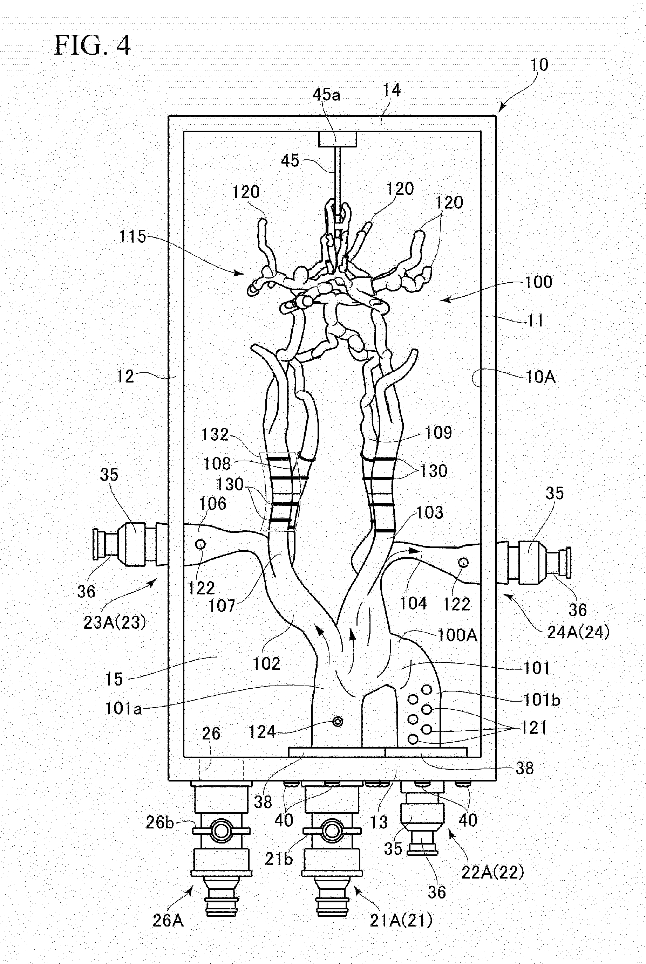

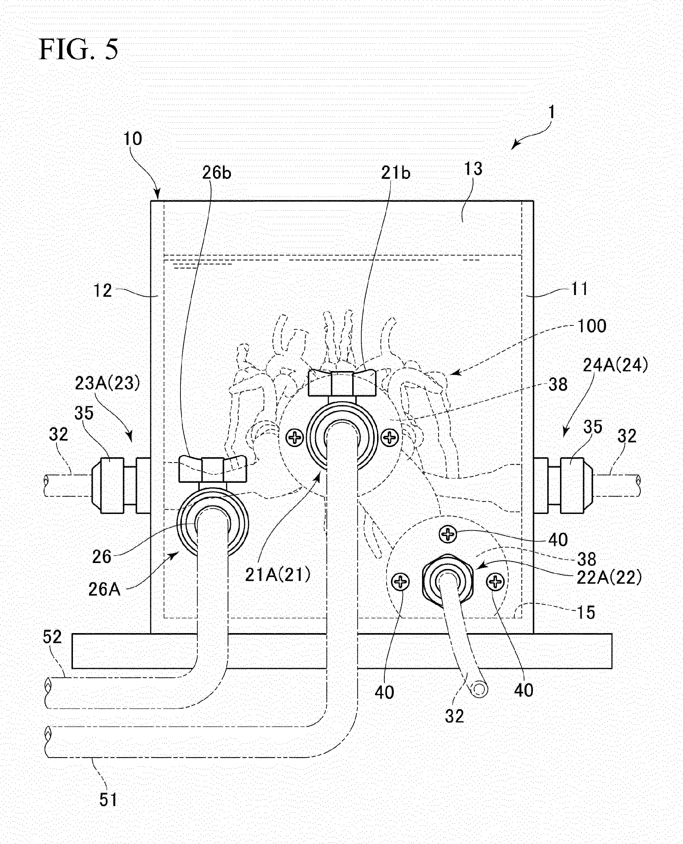

Fig. 1 is a diagram illustrating an embodiment of a catheter simulator according to the present invention, Fig. 2 is a plan view illustrating a container constituting the catheter simulator shown in Fig. 1, Fig. 3 is a diagram illustrating a configuration example of the cerebral blood vessel model, Fig. 4 is a plan view illustrating a state in which the cerebral blood vessel model is installed in the container, and Fig. 5 is a diagram viewing the state in which the cerebral blood vessel model is installed in the container, from one lateral face side.

The catheter simulator 1 according to the present embodiment has a container 10 in which a cerebral blood vessel model 100 as an object of simulation is accommodated, and a pump (pulsatile flow generating pump) 50 that circulates a liquid (mainly water) accommodated in the container 10.

The container 10 is configured as a box body having an approximately rectangular parallelepiped shape including side walls 11 to 14 on four sides and a bottom part 15. In the accommodation part 10A surrounded by the side walls 11 to 14 and the bottom part 15, with the top face opened, a liquid is accommodated through the opening of the top face, and at the same time, the cerebral blood vessel model 100 is detachably held.

The side walls 11 to 14 and the bottom part 15 are produced from a material having a strength capable of stably accommodating and holding the liquid and the cerebral blood vessel model. In addition, it is preferable that the side walls 11 to 14 and the bottom part 15 are formed from a material that is transparent and lightweight and has strength (for example, acryl, polycarbonate, PET, or polystyrene), so that the behavior of the cerebral blood vessel model, the catheter to be inserted from the outside of the container, and the like can be observed by visual inspection during a simulation.

Incidentally, the container 10 may be configured such that the container 10 is formed from a non-transparent material to make the inside visually not recognizable. In this way, even when the inside of the container is visually not recognizable, it is also possible to carry out a simulation of grasping the behavior of the catheter only on the monitor by capturing images with a camera and display the images on a monitor or the like, or by performing fluoroscopy using X-rays and displaying the images on a monitor or the like. That is, the container 10 may be configured so as to allow selection of visual recognition, monitor display checking, or use of X-ray imaging according to the stage and contents of training (even when a transparent material is used, simulation may be performed by putting a cover).

With regard to the shape and the size of the container 10, the shape and the size are not limited as long as the container 10 can stably hold a cerebral blood vessel model that is approximately the same as the cerebral blood vessels of the actual human body. As described above, the elements accommodated in the container 10 are nothing but a cerebral blood vessel model of about the same size as the cerebral blood vessels in the human body and a liquid for stably holding the cerebral blood vessel model, it is possible to miniaturize the container 10. Specifically, the amount of the liquid to be filled in the container 10 can be set to approximately 3 L to 6 L, and with this size of the container 10, there is no wasted space in the place where the simulation is performed, and improvements in the storability and transportability of the catheter simulator 1 can be promoted. In addition, although the upper part of the container 10 is opened, a lid that is openable or closable, or that is detachable, may be provided in the upper part. As a result, when doing preparation or cleanup for training, such as an operation of accommodating a liquid in the accommodation part 10A and an operation of holding the cerebral blood vessel model in the container, the operations can be carried out efficiently through the opening at the top face of the container, while deterioration of visibility caused by waves and reflections on the water surface is prevented.

On the side walls, while the accommodation part 10A is filled with a liquid, the cerebral blood vessel model 100 (see Fig. 3) is held in a floating state (state of being not in contact with the bottom part 15). The cerebral blood vessel model 100 of the present embodiment is configured in a state of imitating the main blood vessel part in the cranium of the human head, and the outer shell thereof (main body 100A) is formed into a shape similar to the actual cerebral blood vessels.

Here, the configuration of the cerebral blood vessel model 100 of the present embodiment will be described with reference to Fig. 3.

As is well known, the blood flow to the brain mainly passes, from the heart via the aorta, through a total of four blood vessels, namely, the left and right carotid arteries (left common carotid artery/right common carotid artery) and the left and right vertebral arteries (left vertebral artery/right vertebral artery) running along the bones of the neck. In the cranium, a large number of blood vessels branch off from these four blood vessels, and the large number of blood vessels are stretched around so as to cover the inside of the brain.

The cerebral blood vessel model 100 shown in Fig. 3 includes an aortic arch 101 composed of an ascending aorta 101a and a descending aorta 101b, through which the blood flow from the heart flows, and three arteries (brachiocephalic artery 102, left common carotid artery 103, and left subclavian artery 104) that branch off in the middle portion of the aortic arch 101. In this case, the brachiocephalic artery 102 branches into a right subclavian artery 106, a right common carotid artery 107, and a right vertebral artery 108, and a left vertebral artery 109 branches off from the left subclavian artery 104. As described above, in the cranium, a large number of blood vessels branch off from the right common carotid artery 107, the left common carotid artery 103, the right vertebral artery 108, and the left vertebral artery 109, and are stretched around so as to cover the inside of the brain (in the cerebral blood vessel model of the present case, the large number of branching blood vessels are indicated as a blood vessel group 115).

The above-described cerebral blood vessel model 100 is held in the inner surface portion of the side walls of the container 10. In the present embodiment, the cerebral blood vessel model 100 is held at the end of the ascending aorta 101a and the end of the descending aorta 101b, and is further held at the end of the right subclavian artery 106 and the end of the left subclavian artery 104 (end of each blood vessel), so that the cerebral blood vessel model 100 as a whole is held in a well-balanced manner in a state of floating from the bottom part. For this reason, a holding means for holding the end of each blood vessel is provided at each of the above-described blood vessels and the corresponding positions on the side walls 11, 13, and 14 of the container 10.

The configuration of the holding means will be described below.

The holding means of the present embodiment includes: a first holding part 21 that holds the end of the ascending aorta 101a; a second holding part 22 that holds the end of the descending aorta 101b; a third holding part 23 that holds the end of the right subclavian artery 106; and a fourth holding part 24 that holds the end of the left subclavian artery 104. In this case, the first holding part 21 that holds the end of the ascending aorta 101a is connected to a side wall of the container 10 at an approximately right angle. In the cerebral blood vessel model 100, as a curve-shaped aortic arch 101 is disposed inside the container 10, a pulsatile flow flowing in through the ascending aorta side 101a is not linear and flows along the curve-shaped blood vessel. As a result, this brings a pulsatile flow to the left and right internal carotid arteries and vertebral arteries, and imaging of only the blood vessels in either the left or right cerebral hemisphere is realized, as in actual clinical settings. In this case, it is also structurally possible to dispose the aortic arch portion on the outside of the container. However, a plurality of blood vessels branch off from the aortic arch, and there may occur a decrease in usability due to difficulties in handling, an increase in the risk of damage, and at the time of observing by X-ray fluoroscopy, a deviation between the observed image and the actual clinical image caused by reflected glare of the container.

On the other hand, it is also possible to exclude the aortic arch itself from the configuration; however, in that case, as described above, a pulsatile flow from the pump flows linearly into each intracranial blood vessel and interferes with the balance of the blood flow. That is, in order to meet the requirements of both the usability and the realization of a well-balanced blood flow close to the actual clinical settings, the present configuration is most suitable.

Incidentally, a heart can be connected to the aortic arch in the same way as in the actual human body; however, in that case, the simulator itself becomes large, which leads to a decrease in usability and an increase in the risk of damage. By specializing in cerebrovascular catheter maneuvers, the present simulator is intended to enable medical professionals to easily perform training for maneuvers when necessary, and these needs can be realized by simplifying the configuration.

In the above-described configuration, the first holding part 21 has a function as an introduction path for introducing a liquid into the cerebral blood vessel model (liquid introduction port). For this reason, the portion where the first holding part 21 is provided has a through-hole (liquid introduction port) 21a formed in the side wall 13, and at this through-hole 21a, a cylindrical-shaped connection part (known one-touch connector) 21Aprotruding from the side wall 13 to the outside is coaxially provided. An introduction tube 51 from the pump 50 is to be connected to this connection part 21A (see Fig. 1).

In addition, on the side wall 13 of the container 10, a discharge port (through-hole) 26 for discharging the liquid inside the accommodation part 10Ato the outside is provided adjacently to the position where the first holding part 21 is formed. At this discharge port 26, a cylindrical-shaped connection part (known one-touch connector) 26A protruding from the side wall 13 to the outside is coaxially provided, and a discharge tube 52 to the pump 50 is to be connected to the connection part 26A (see Fig. 1). This discharge port may be configured to be connected to the cerebral blood vessel model 100; however, when the discharge port is not connected to the cerebral blood vessel model 100, the installation operation can be easily performed.

Incidentally, it is preferable that valves for opening and closing (detailed configurations are not shown in the diagram) are installed in the connection parts 21A and 26A. The flow path of each connection part can be opened or closed by rotatively manipulating manipulation parts 21b and 26b provided in the connection parts 21A and 26A, respectively. These valves for closing prevent the liquid inside the accommodation part 10A from escaping to the outside, when the pump 50 is detached from the container 10 after completion of a simulation, by rotatively manipulating the manipulation parts 21b and 26b to close the flow path.

The second holding part 22 has a function as an introduction part (catheter introduction port) for introducing a catheter from the outside of the container 10 into the cerebral blood vessel model 100. For this reason, the portion where the second holding part 22 is provided has a through-hole (catheter introduction port) 22a formed in the side wall 13, and at this through-hole 22a, a cylindrical-shaped introduction connector 22A protruding from the side wall 13 to the outside is coaxially provided. An introduction tube (tube) 32 for the catheter is to be connected to this introduction connector 22A.

The introduction tube 32 for catheter has a catheter introduction terminal (sheath) 32a at the distal end part so that the introduction tube 32 has a function (valve function) of preventing the liquid filled in the introduction tube 32 from leaking to the outside, and also has a structure that enables a trainee to introduce a catheter into the introduction tube 32 and pull out the catheter therefrom.

The introduction connector 22Ahas a connection mechanism that can be manipulated from the outside of the container 10. This connection mechanism has, for example, a structure in which the introduction tube 32 can be fixed or released by plugging in the introduction tube 32 and rotating a manipulation member (nut) 35, so that attachment and detachment of the introduction tube 32 can be easily manipulated. Incidentally, when the introduction tube 32 is not plugged in (not used), the opening is closed with a plug member 36 (see Fig. 2).

In addition, in the present embodiment, the third holding part 23 and the fourth holding part 24 also have a function as an introduction part (catheter introduction port) for introducing a catheter from the outside of the container 10 into the cerebral blood vessel model 100, similarly to the above-described second holding part 22. These have through-holes (catheter introduction ports) 23a and 24a formed in the side walls 12 and 11 at the portions where the respective holding parts 23 and 24 are provided, similarly to the second holding part 22, and at these through-holes 23a and 24a, cylindrical-shaped introduction connectors 23A and 24A protruding from the side wall to the outside are coaxially provided. An introduction tube (tube) 32 for catheter is to be connected to these introduction connectors 23A and 24A, similarly to the above-described introduction connector 22A. Such a catheter introduction port may be provided in either one of the third holding part 23 and the fourth holding part 24.

The cerebral blood vessel model 100 as shown in Fig. 3 is mounted and held by the above-described first holding part to the fourth holding part. Here, the mode of holding the cerebral blood vessel model according to the present embodiment will be described.

As described above, the end of the ascending aorta 101a is held by the first holding part 21, and the end of the descending aorta 101b is held by the second holding part 22. In this case, the entirety of the cerebral blood vessel model 100 of the present embodiment is formed from hard resins, and a hard flange 38 is integrally formed at each of the ends of the ascending aorta 101a and the descending aorta 101b. In addition, the end openings 101e and 101f of the ascending aorta 101a and the descending aorta 101b are formed to have sizes that match the sizes of the through-holes 21a and 22a formed in the side wall 13, respectively. When each flange 38 is closely attached to the inner face of the side wall 13 of the container, and a screw 40 is inserted from the outside of the container into a screw hole 38a formed in each flange 38, the flange 38 is closely attached and fixed to the side wall inner face. That is, the end region of the ascending aorta 101a is in a state of being connected to the side wall 13 of the container at an approximately right angle. As a result, the ends of the ascending aorta 101a and the descending aorta 101b are held (fixed) on the inner face of the side walls of the container by the first holding part 21 and the second holding part 22, in a state of communicating with the through-holes 21a and 22a.

In addition, the end of the right subclavian artery 106 is held by the third holding part 23, and the end of the left subclavian artery 104 is held by the fourth holding part 24. In this case, a cylindrical part 23b or 24b protruding to the inside of the container is provided at each of the holding part 23 or 24, coaxially with the through-hole 23a or 24a, and a male screw part 23c or 24c is formed on the outer peripheral surface of each of the cylindrical parts. On the other hand, a female screw part 106a is formed at the open end of the right subclavian artery 106, and similarly, a female screw part (not shown in the diagram) is also formed at the open end of the left subclavian artery 104. As a result, by screwing the respective screw parts together, the right subclavian artery 106 and the left subclavian artery 104 are held (fixed) on the inner faces of the side walls of the container by the third holding part 23 and the fourth holding part 24.

The holding means of the present embodiment further has a locking member 45 that holds the cerebral blood vessel model 100 on the inner face of the container 10. This locking member 45 has a function of stabilizing the holding state by locking the cerebral blood vessel model and is installed on the side wall 14, where no holding part is provided, among the side walls. The locking member 45 is formed into a rod shape protruding to the inside of the container so that the locking member 45 can lock the blood vessel group 115 of the cerebral blood vessel model 100. As a result, the cerebral blood vessel model 100 is held on all of the inner faces of the side walls 11 to 14 of the container 10, and therefore, a stable holding state is obtained. In this case, the locking member 45 is preferably configured such that, for example, a flat pedestal 45a is installed on the side wall 14, and the locking member 45 can be attached to or detached from the pedestal. By configuring the locking member 45 to be attachable and detachable in this way, the operation is not interfered with when the cerebral blood vessel model is mounted or detached.

It is preferable to adhere an auxiliary plate for reinforcing strength at the portion where the holding means (holding part) is provided. When strength reinforcement is promoted by the auxiliary plate, the entire container can be made lightweight as compared with the case of increasing strength by making the entire side walls thicker. Incidentally, when the visibility of a passing catheter or the like is lowered by sticking the auxiliary plate to the side wall, the thickness of the side wall may be increased only on the side where strength increase is required. Furthermore, it is preferable that the side walls are formed into a flat plate shape without surface unevenness, and as a result, reflection of light does not occur so that an increase in visibility of the inside can be promoted.

When the cerebral blood vessel model 100 is placed inside the container 10 in a state of being filled with a liquid, and the liquid is circulated by a pump 50, the input from the pump 50 is introduced into the cerebral blood vessel model through the first holding part 21 (connection part 21A). For this reason, it is necessary to form an opening part for draining the liquid in the outer shell 100A of the cerebral blood vessel model 100.

In addition, as described above, in the actual human body, about 15% of the blood flow sent from the heart circulates in the brain, while about 90% circulates in the body. On the occasion of forming an opening part in the outer shell that constitutes the cerebral blood vessel model, it is possible to adjust the balance of the liquid circulating the blood vessels in the brain by taking the position of formation, size, and the like of the opening part into consideration, and it is possible to perform a simulation of the catheter maneuvers in a state close to the blood flow of the actual human body.

When a liquid is introduced into the cerebral blood vessel model 100 through the pump 50, as shown by the arrow in Fig. 4, the liquid flows through the ascending aorta 101a to the brachiocephalic artery 102, the left common carotid artery 103, the left subclavian artery 104, and the descending aorta 101b. In addition, the liquid that has flowed through the brachiocephalic artery 102 flows to the right subclavian artery 106, the right common carotid artery 107, and the right vertebral artery 108, and the liquid that has flowed through the left subclavian artery 104 flows to the left vertebral artery 109. Then, the liquid that has flowed through the right common carotid artery 107, the right vertebral artery 108, the left common carotid artery 103, and the left vertebral artery 109 flows directly to the blood vessel group 115. By forming opening parts 120 at the distal ends of some of the blood vessel group 115, the liquid introduced through the ascending aorta 101a generates a flow circulating inside the cerebral blood vessels, and a blood flow equivalent to the cerebral blood vessels of the actual human body is reproduced. For this reason, the liquid discharged into the container through the opening parts 120 flows through the discharge port 26 of the container into the discharge tube 52 and is circulated via the pump 50.

In the present embodiment, a plurality of opening parts 121 are formed on the root side of the descending aorta 101b connected to the ascending aorta 101a, so as to reproduce the same flow as the blood flow flowing through the thorax, abdomen, and lower limbs in the actual human body. That is, when an opening part 121 is not formed in the descending aorta 101b, the liquid flow returns to the superior aorta side at this portion, thereby a flow that does not originally exist in the human body is generated, and an appropriate simulation cannot be achieved.

Furthermore, in the present embodiment, one opening part 122 is also formed at each of the ends of the right subclavian artery 106 and the left subclavian artery 104.

By forming opening parts 122 at both the ends of the left and right subclavian arteries 104 and 106 in this way, the flow rate can be controlled in a well-balanced manner, and at the same time, when contrast imaging is performed from the aorta, the contrast medium is appropriately discharged without being accumulated in the blood vessel. Incidentally, with regard to the opening part 122, it may be configured such that the opening part 122 is formed in either one of the left and right subclavian arteries 104 and 106.

With regard to the above-described opening parts 120, 121, and 122, it is possible to realize the flow of the blood flow in the actual human body and the blood flow balance by optimizing the size, the position of formation, and the number of formations of the opening parts. In this case, when an opening part is formed on the path of the catheter to be inserted, there is a possibility that the catheter may jump out of the blood vessel, and therefore, it is preferable not to form an opening part on the insertion path. In addition, in the present invention, the opening parts 121 and 122 are formed on the top face side of each blood vessel as shown in Fig. 4; however, the direction is not limited. For example, when the opening parts are formed on the bottom face side, ripples generated on the water surface during the simulation can be suppressed to contribute to visibility, and at the same time, the appearance can be improved.

In addition to the above-described opening parts that control the flow rate, it is preferable to form air holes for releasing air. When setting up for the first time, air may enter the cerebral blood vessel model, and when a liquid is circulated inside the cerebral blood vessel model in this state, there is a possibility that a dead air space may occur at the highest position and cause a problem of hindering visibility. Therefore, when the cerebral blood vessel model 100 is set up, an air hole 124 is formed at the highest position (in the present embodiment, the highest position in the superior aorta 101a), and the air accumulated during liquid circulation is allowed to be released.

As described above, by forming opening parts 120, 121, and 122 that control the flow rate of the liquid to be introduced in the outer shell 100A of the cerebral blood vessel model 100, it is possible to easily reproduce the blood flow flowing through the cerebral blood vessels with a simple configuration. On the occasion of adjusting the liquid flow, it is conceivable to provide adjustment valves for adjusting the flow rate in the portion through which the liquid passes; however, such a valve mechanism has a complicated structure, and the adjustment manipulation becomes troublesome. In addition, since the adjustment valves are reflected in the image under X-ray fluoroscopy, performing a simulation under imaging close to the actual clinical settings is hindered. In a simulation of catheter maneuvers utilizing the above-described cerebral blood vessel model, since fine adjustment of the blood flow is not necessarily essential, it is possible to set up the cerebral blood vessel model easily and conveniently without providing adjustment valves, by optimizing the size and position of the opening parts.

The above-described cerebral blood vessel model 100 can be produced by integrally molding a transparent hard resin material (for example, polyurethane, an epoxy resin, an acrylic resin, a polycarbonate resin, an unsaturated polyester, a vinyl chloride resin, or polyethylene terephthalate) by using a 3D-printer. By forming the cerebral blood vessel model using a hard material in this way, it is possible to stably maintain a fixed state in the container accommodating water, and a simulation according to actual catheter manipulation can be performed without having the cerebral blood vessel model move around unnaturally.

Alternatively, it is also possible to form the cerebral blood vessel model using a resin material having elasticity close to the blood vessels of the actual human body, for example, PVA (polyvinyl alcohol), polyurethane, an epoxy resin, unsaturated polyester, a phenol resin, silicon, a material similar to one of these, and a thermosetting resin or thermoplastic resin other than those, singly or in combination of multiple materials. When the cerebral blood vessel model is formed using an elastic resin material, it is possible to appropriately modify each of the holding parts 21 to 24 constituting the above-described holding means. For example, the cerebral blood vessel model may be held by forming cylindrical-shaped protrusions that protrude from the side walls toward the inside and communicate with the outside, and plugging the opening of the ends of blood vessels into the outer periphery of these protrusions.

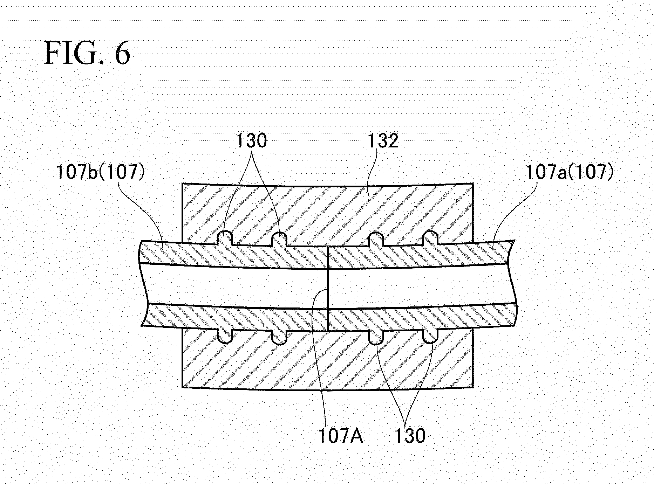

It is preferable that each blood vessel of the above-described cerebral blood vessel model 100 is integrally formed without seams; however, each blood vessel portion (at any position) may be configured such that the blood vessels can be separated by, for example, a push-in system. In this case, for example, as shown in Fig. 3 and Fig. 4, a stopper part (for example, a ring-shaped locking part 130) is formed on the outer peripheral surface in some of the blood vessels (left common carotid artery 103, left vertebral artery 109, right common carotid artery 107, right vertebral artery 108, and the like), and when the blood vessel (blood vessel part) of this portion is made exchangeable by a plug-in system, the inserted blood vessel part becomes less likely to fall out due to the locking part 130, which makes it possible to perform a stable simulation. Specifically, for example, when the right common carotid artery 107 is cut and made attachable and detachable as a blood vessel part, as shown in Fig. 4 and Fig. 6, the blood vessel parts can be made freely attachable and detachable by putting a soft joint 132 on either one of the blood vessel parts 107a and 107b in which the locking part 130 is formed, plugging the other blood vessel part therein in this state, and putting the end faces 107A against each other.

Such blood vessel parts are not limited in terms of the material, such as a soft resin material; and for example, it is possible to separately produce a blood vessel part having a lesioned part similar to a lesion in the actual human body, such as a stenosis part or a cerebral thrombosis part, so that this portion can be made attachable and detachable. That is, by making some of the blood vessel portions of the cerebral blood vessel model 100 separable, there is no need to detach the main body itself from the container 10, it is possible to attach blood vessels having a plurality of shapes, and it is possible to install a lesioned part for which practice is needed, and to perform an appropriate simulation.

As described above, for the cerebral blood vessel model 100 placed in the container 10, it is possible to allow a catheter to reach a lesioned part with a sensation close to that of a cerebral blood vessel, and to train a catheter manipulation such as occluding or dilating the lesioned part. In this case, by producing a cerebral blood vessel model 100 using a transparent or translucent material, a trainee can observe the movement of an inserted catheter, a guide wire, and other devices directly by visual inspection, and at the same time, can visually recognize the behavior exhibited by an injected agent injected through the catheter. That is, it is possible to simulate catheter manipulation while linking the hand manipulation and the movement of the distal end of the catheter. In addition, even when a cerebral blood vessel model 100 is produced with a material that can be visually recognized by a trainee, the container 10 may be covered with a cover or the like so that the cerebral blood vessel model cannot be seen, or when X-ray fluoroscopy is performed and the data is displayed on a monitor or the like, it is also possible to grasp the behavior of the catheter only on the monitor.

The catheter simulator of the above-described embodiment is configured such that a pulsatile flow from the pump 50 flows into the ascending aorta 101a of the cerebral blood vessel model through the first holding part 21. When an actual human anatomical form is reproduced as it is, the ascending aorta 101a and the first holding part 21 are connected to the side wall 13 of the container 10 not at right angles but from an oblique direction. In such a connection form, the pulsatile flow flowing through the brachiocephalic artery 102, the left common carotid artery 103 side and the left subclavian artery 104 side is likely to be balanced; however, the capacity of the container 10 may increase, impairing usability. In addition, the structure becomes complex, and there is a disadvantage that the production process becomes complicated.

For this reason, it is preferable that the ascending aorta 101a and the first holding part 21 are disposed at right angles to the side wall 13 of the container 10 as described above, and it is preferable that the pulsatile flow of the pump 50 flows into the side wall 13 of the container at an approximately right angle. However, the pulsatile flow that has flowed in from the ascending aorta side 101a is not linear and flows along a curve-shaped blood vessel, and in the disposition mode of the cerebral blood vessel model shown in Fig. 4, there is a possibility that the pulsatile flow flowing to the brachiocephalic artery 102 side may become larger than the pulsatile flow flowing to the left common carotid artery 103 side and the left subclavian artery 104 side (the blood flow distribution to intracranial blood vessels may be unbalanced). For this reason, for the liquid flowing from the pump 50 into the cerebral blood vessel model, it is preferable to provide a flow rate adjustment means in the end region of the ascending aorta, which serves as the inflow portion for the liquid.

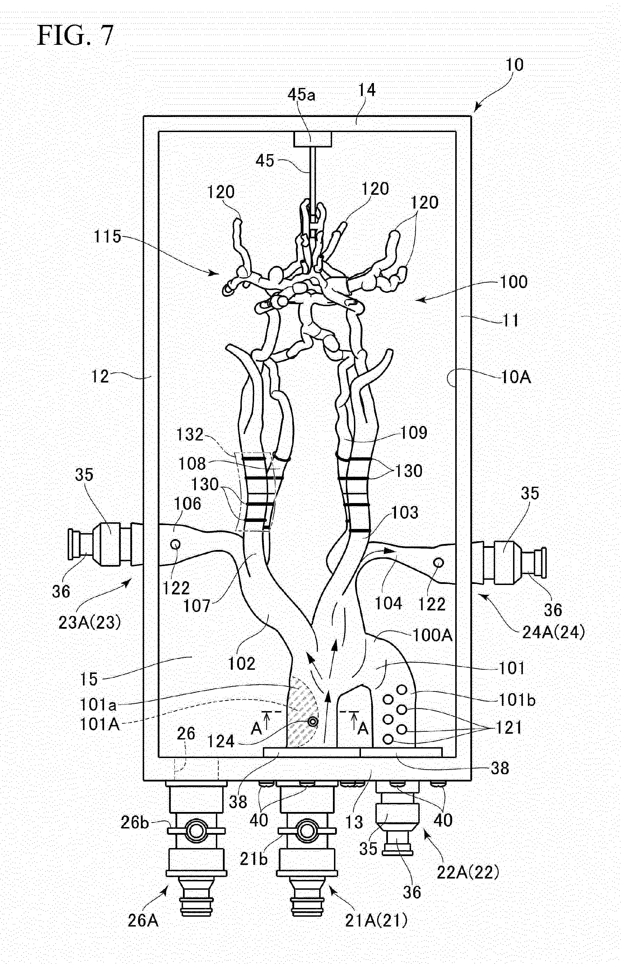

Fig. 7 is a diagram showing a first example of the flow rate adjustment means for the liquid flowing into the cerebral blood vessel model.

In this flow rate adjustment means, a convex-shaped protrusion part 101A is formed on the inner wall of the inflow part from the pump, specifically, the distal end part of the ascending aorta 101a connected to the connection part 21A of the first holding part 21. Such a protrusion part 101Ahas a function of reducing the pulsatile flow flowing to the brachiocephalic artery 102 side, increasing the pulsatile flow flowing to the left common carotid artery 103 side and the left subclavian artery 104 side, and adjusting the pulsatile flows flowing to both sides to be uniform (approximately uniform).

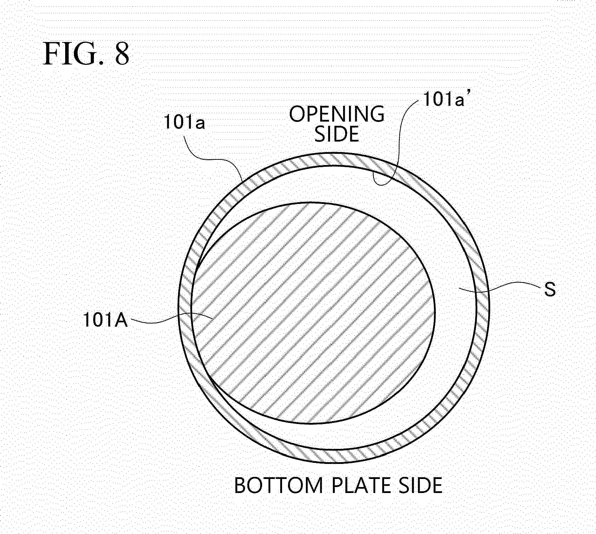

There is a possibility that the convex-shaped protrusion part 101A may be recognized as protrusions that cannot be seen in the actual clinical settings when viewed under X-ray fluoroscopy. However, as shown in the cross-sectional view of Fig. 8, when a gap S is intentionally provided between the bottom plate side and the opening side of the protrusion part 101A and the inner wall 101a' of the ascending aorta 101a connected to the connection part 21A, it is possible to bring the X-ray fluoroscopic image closer to that of the actual clinical settings while maintaining the distribution balance of the pulsatile flows.

It is possible to obtain an optimal liquid flow distribution for the left and right intracranial blood vessels (blood vessel group and the like), by forming such a protrusion part 101A. Incidentally, the method for forming the protrusion part 101A is not particularly limited, and for example, the protrusion part 101A may be formed integrally on the inner wall of the distal end part of the ascending aorta 101a, or a protrusion part may be formed as a separated body and then attached to the inner wall of the ascending aorta 101a by adhesion or the like. In addition, the position of formation, size, and the like of the protrusion part can be appropriately modified.

Furthermore, an air hole 124 is formed in the ascending aorta 101a so as to allow air to escape during setting as described above; however, with regard to this air hole 124, it is preferable to adopt a one-way valve so as to prevent inflow of air into the cerebral blood vessel model from the outside.

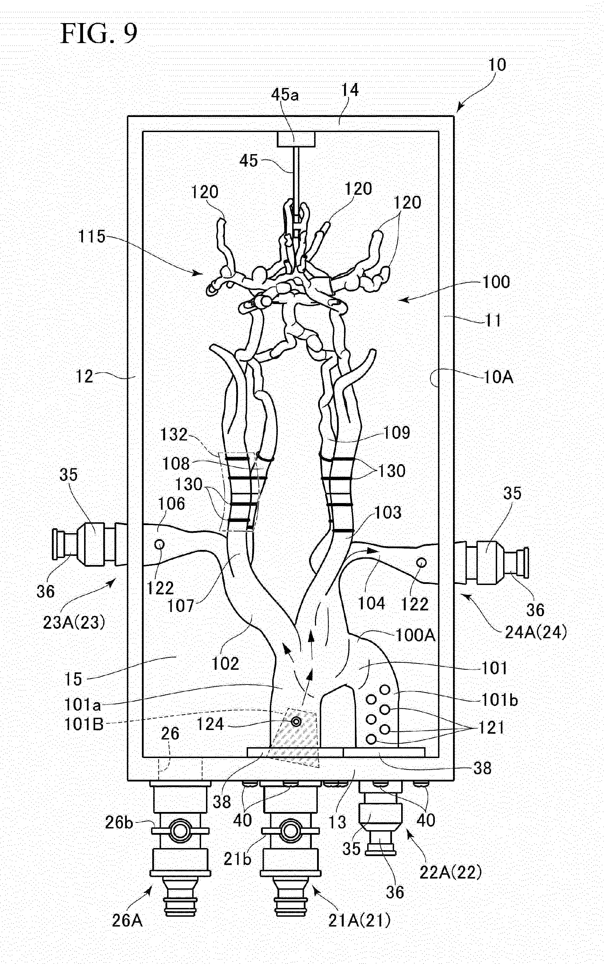

Fig. 9 is a diagram illustrating a second example of the flow rate adjustment means for the liquid flowing into the cerebral blood vessel model.

This configuration example shows an example in which a member for flow rate adjustment is disposed not on the inner wall of the end region of the ascending aorta 101a but within the opening region of the ascending aorta 101a. This member for flow rate adjustment is composed of a nozzle 101B disposed within the opening 101e of the flange 38 (see Fig. 3), and the distal end side thereof is directed toward to the left common carotid artery 103 side. Even with such a configuration of disposing a nozzle, it is possible to obtain an optimal liquid flow distribution for the left and right intracranial blood vessels, as is the case of the above-described protrusion part 101A. Incidentally, the direction of the nozzle 101B and the direction of the connection portion of the ascending aorta 101a can be appropriately modified. For example, by reversing the direction of the nozzle 101B shown in Fig. 9, while bending the proximal end portion of the ascending aorta 101a toward the descending aorta 101b side and connecting the proximal end portion to the connection part 21A, a blood flow similar to that of the actual heart can be realized, and an optimal liquid flow distribution can be obtained.

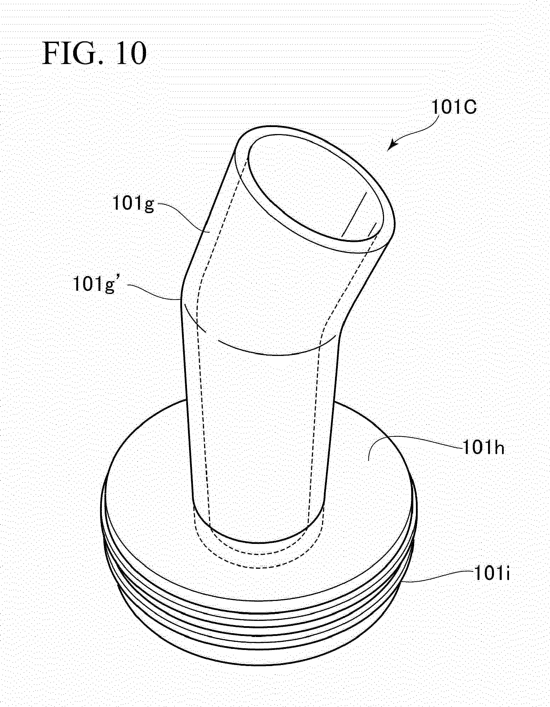

In addition, Fig. 10 is a diagram illustrating a third example of the flow rate adjustment means for the liquid flowing into the cerebral blood vessel model. A nozzle 101C of this configuration example is configured such that an intermediate part 101g' of a cylindrical-shaped nozzle 101g is formed to be bent, and a flange 101h is integrally formed at the proximal end of this nozzle 101g. On the outer surface of the flange 101h, a threaded part 101i to be screwed into the screw part formed at the opening 101e of the flange 38 shown in Fig. 3 is formed, and the direction of the pulsatile flow guided into the ascending aorta 101a is adjusted by screwing the two together.

According to such a nozzle configuration, it is possible to adjust the flow rate only by connecting the ascending aorta 101a of the cerebral blood vessel model 100 to the connection part 21A, and assembling and flow rate adjustment can be easily carried out.

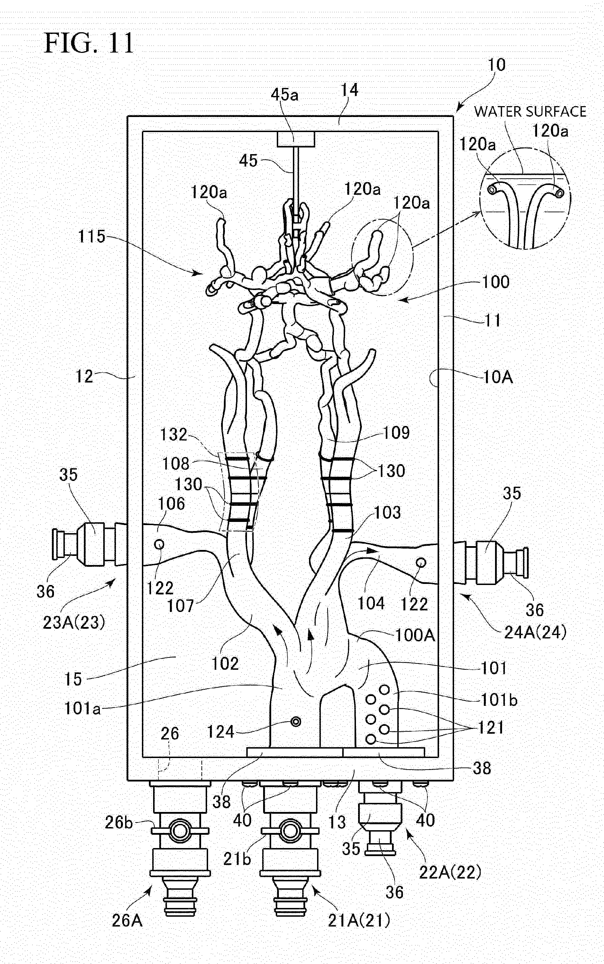

Fig. 11 is a diagram illustrating a modification example of the blood vessel group 115 of the cerebral blood vessel model 100.

As described above, in the cerebral blood vessel model 100, opening parts 120, 121, and 122 are formed at appropriate places so that a flow similar to that of the actual blood can be reproduced, and an appropriate simulation can be performed. In this case, when the opening parts are formed such that the distal end side of the blood vessel group 115 face upward, the liquid flow bursts out upward, causing the liquid surface (water surface) to sway or the liquid flow to be jetted, and there is a possibility that an appropriate simulation may be interfered with. As shown in Fig. 11, it is possible to stabilize the liquid surface during a simulation by forming a bent part 120a such that the openings at the distal ends of the blood vessel group 115 face downward with respect to a horizontal surface (liquid surface). In addition, it is also possible to float a top plate on the liquid surface instead of these. Since a top plate suppresses shaking of the liquid surface and also prevents deterioration of visibility caused by ripples, it is desirable to use the top plate regardless of the direction of the opening parts at the distal ends of the blood vessel group 115.

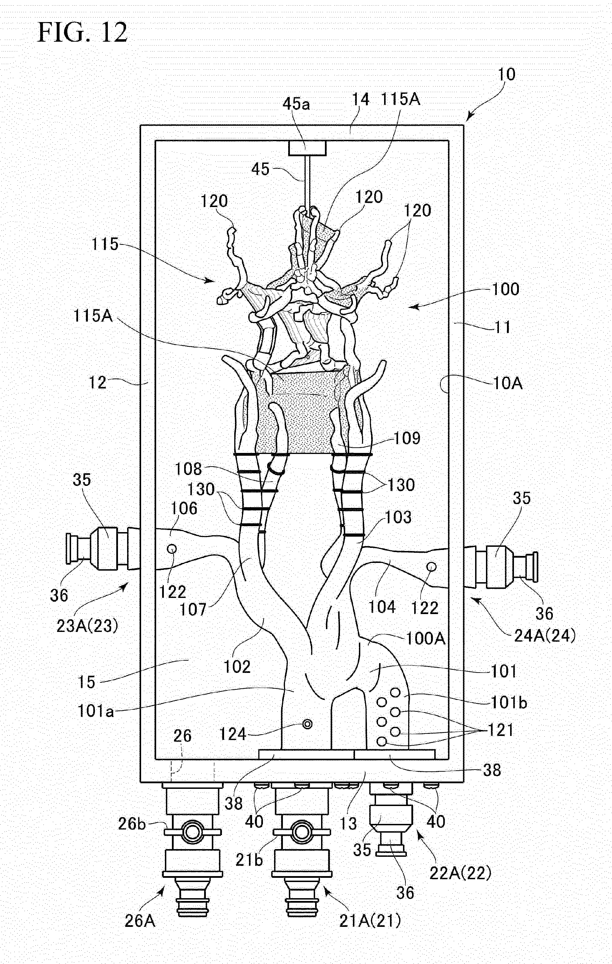

Fig. 12 is a diagram illustrating a second modification example of the blood vessel group 115 of the cerebral blood vessel model 100.

As described above, by forming the cerebral blood vessel model 100 from a hard material, it is possible to stably maintain the fixed state in a container accommodating water, and it is possible to perform a simulation according to an actual catheter manipulation without having the cerebral blood vessel model move unnaturally. In this case, when the blood vessel group 115 is formed from a hard material, there is a possibility of damage such as breakage or cracking, when the cerebral blood vessel model is set in the container 10, removed from the container, or transported. Particularly, since the blood vessel group 115 has fine diameters, the blood vessel group 115 is easily damaged when the blood vessel group 115 comes into contact with another object, or stress is applied thereto.

For this reason, it is preferable that a large number of joining pieces 115A are integrally formed together with the blood vessel group such that the blood vessels of the blood vessel group 115 are integrally joined (integrated). The joining pieces 115A are formed into a thin plate shape and are installed in the blood vessel group so as to integrally join any blood vessels of the blood vessel group 115. These joining pieces 115A may integrally join at least some of the blood vessel group 115.

When such joining pieces 115A are formed from a transparent material as in the case of the blood vessel group, an actual simulation is not interfered with, and a handling manipulation can be easily performed.

With regard to the cerebral blood vessel model 100, in order to obtain an image close to the actual clinical settings under X-ray fluoroscopy, it is desirable that the joining pieces 115A are not projected in the image. Since projection of X-rays in angiography is carried out from a certain direction toward the opposite direction across the subject, even when the joining pieces 115A have a planar structure, the joining pieces 115Atend to be easily recognized as opaque objects when the joining pieces 115A are in a direction perpendicular to the projection direction of X-rays. Therefore, it is desirable that the joining pieces 115A have a curved structure rather than a planar structure. In addition, in a structure of a curved surface, air is easily collected; however, this can be solved by providing a hole for releasing air in a portion corresponding to the ceiling of the curved surface. Furthermore, with regard to the cerebral blood vessel model, since images are often captured at an angle in a direction perpendicular to the bottom face of the container 10, it is desirable that the joining pieces 115A are formed in a plane approximately parallel to the bottom face of the container 10.

Exemplary embodiments of the catheter simulator and the cerebral blood vessel model according to the present invention have been described; however, the present invention is not limited to the above-described embodiments, and various modifications can be applied to the extent that does not deviate from the purpose of the present invention. For example, as long as the cerebral blood vessel model 100 is held inside a container 10 filled with a liquid, a liquid flow similar to the blood flow in the actual human body is generated inside the blood vessels, and a manipulation of catheter insertion in such a holding state can be performed, the configuration (fixing method) of the holding parts of the model is not limited. In addition, it is possible to appropriately modifying the configuration of the blood vessels of the cerebral blood vessel model 100, and particularly the blood vessel group 115. Furthermore, it is also possible to appropriately modify the configuration of the holding means for holding the cerebral blood vessel model inside the container. For example, a configuration including a mounting pedestal for mounting the cerebral blood vessel model 100 may also be adopted.

### EXPLANATIONS OF LETTERS OR NUMERALS

1 CATHETER SIMULATOR
10 CONTAINER
21 TO 24 FIRST HOLDING PART TO FOURTH HOLDING PART (HOLDING MEANS)
45 LOCKING MEMBER (HOLDING MEANS)
50 PUMP
100 CEREBRAL BLOOD VESSEL MODEL
100A OUTER SHELL
115 BLOOD VESSEL GROUP
120, 121, 122 OPENING PARTS

## Claims

1. A catheter simulator comprising:
a container capable of accommodating a liquid in an accommodation part surrounded by side walls and a bottom part;
a cerebral blood vessel model held in the container in a state of accommodating a liquid;
a pump connected to the container and circulating the liquid inside the cerebral blood vessel model held therein; and
a holding means provided on the side walls of the container and the cerebral blood vessel model and holding the cerebral blood vessel model in a state of having the container filled with the liquid,
wherein the holding means includes a first holding part holding an end of an ascending aorta of the cerebral blood vessel model, and a second holding part holding an end of a descending aorta of the cerebral blood vessel model,
the first holding part includes a liquid introduction port for introducing a liquid into the cerebral blood vessel model in the state of being held,
the second holding part includes a catheter introduction port for introducing a catheter into the cerebral blood vessel model in the state of being held, and
an opening part for controlling the balance of the liquid circulating inside the cerebral blood vessel model by discharging the liquid to be introduced by the pump, is formed on an outer shell constituting the cerebral blood vessel model.

2. The catheter simulator according to claim 1, wherein the opening part is provided in the descending aorta held by the second holding part.

3. The catheter simulator according to claim 1 or 2, wherein the opening part is provided at an end of a blood vessel constituting the cerebral blood vessel model.

4. The catheter simulator according to claim 3, wherein the opening part provided at the end of a blood vessel is formed to face downward with respect to the liquid surface of the liquid accommodated in the container.

5. The catheter simulator according to claim 1, wherein the cerebral blood vessel model is held in a state of being not in contact with the bottom part of the container.

6. The catheter simulator according to claim 1, wherein in the cerebral blood vessel model, a portion of an aortic arch is disposed on the inner side of the container.

7. The catheter simulator according to claim 1, wherein the holding means has a third holding part and a fourth holding part holding an end of a right subclavian artery and an end of a left subclavian artery of the cerebral blood vessel model, respectively.

8. The catheter simulator according to claim 7, wherein the opening part is formed in either one or both of the right subclavian artery and the left subclavian artery.

9. The catheter simulator according to claim 7 or 8, wherein a catheter introduction port for introducing a catheter into the cerebral blood vessel model in the state of being held is provided in either one or both of the third holding part and the fourth holding part.

10. The catheter simulator according to claim 1, wherein the holding means includes a flange formed integrally with a blood vessel end of the cerebral blood vessel model, and
the holding means holds the cerebral blood vessel model inside the container by closely attaching and fixing the flange to the inner face of the container.

11. The catheter simulator according to claim 1, wherein the holding means includes a female screw part formed integrally with the inner face of a blood vessel end of the cerebral blood vessel model, and
the holding means holds the cerebral blood vessel model inside the container by screwing the female screw part together with a male screw part formed on the outer peripheral surface of a cylinder part protruding at the inner face of the container.

12. The catheter simulator according to claim 1, wherein the holding means has a locking member provided on the inner face of the container and locking the cerebral blood vessel model.

13. The catheter simulator according to claim 1, wherein a discharge port for discharging the liquid in the container and being connected to the pump is provided on a side face of the container.

14. The catheter simulator according to claim 1, wherein some of the blood vessels of the cerebral blood vessel model constitute a lesioned part, and the lesioned part is configured to be attachable and detachable as an attachable and detachable blood vessel part.

15. The catheter simulator according to claim 1, wherein the end region of the ascending aorta of the cerebral blood vessel model is connected to a side wall of the container at an approximately right angle.

16. The catheter simulator according to claim 1, wherein a flow rate adjustment means for adjusting the liquid flow distribution for the left and right intracranial blood vessels is provided in the end region of the ascending aorta of the cerebral blood vessel model.

17. The catheter simulator according to claim 16, wherein the flow rate adjustment means is a convex-shaped protrusion part formed on the inner wall of the distal end part of the ascending aorta.

18. The catheter simulator according to claim 16, wherein the flow rate adjustment means is a nozzle disposed within the opening region of the ascending aorta.

19. The catheter simulator according to claim 17, wherein a gap is provided between the bottom plate side as well as the opening side of the convex-shaped protrusion part formed on the inner wall of the distal end part of the ascending aorta and the inner wall of the ascending aorta.

20. The catheter simulator according to claim 1, wherein the first holding part of the container is disposed on a side wall of the container at an approximately right angle.

21. The catheter simulator according to claim 1, wherein a pulsatile flow of the pump flows in at an approximately right angle with respect to a side wall of the container.

22. A cerebral blood vessel model held in a container accommodating a liquid and used when catheter maneuvers are practiced in a state of circulating a liquid therein,
wherein an opening part for controlling the balance of the circulating liquid is formed in an outer shell constituting the cerebral blood vessel model.

23. The cerebral blood vessel model according to claim 22, wherein the opening part is formed at an end of a blood vessel constituting the cerebral blood vessel model.

24. The cerebral blood vessel model according to claim 22, wherein the opening part is formed at at least one or more of a descending aorta, an end of a right subclavian artery, and an end of a left subclavian artery constituting the cerebral blood vessel model.

25. The cerebral blood vessel model according to claim 22, wherein the outer shell constituting the cerebral blood vessel model includes a hard part, and
the cerebral blood vessel model is held in the container using the hard part.

26. The cerebral blood vessel model according to claim 25, wherein at least some of the blood vessel group constituting the cerebral blood vessel model are integrally joined by joining pieces formed into a thin plate shape.

27. The cerebral blood vessel model according to claim 26, wherein at least one of the joining pieces has a curved structure.

28. The cerebral blood vessel model according to claim 26 or 27, wherein at least one of the joining pieces has a hole for releasing air.

29. The cerebral blood vessel model according to claim 22, wherein some of the blood vessels constituting the cerebral blood vessel model are freely attachable and detachable as blood vessel parts.
